# EUROPÄISCHE PATENTSCHRIFT

(11) **EP 2 679 255 B1**
(45) Veröffentlichungstag und Bekanntmachung des Hinweises auf die Patenterteilung: **15.02.2017**
(21) Anmeldenummer: 13003055.4
(22) Anmeldetag: 14.06.2013
(51) Int. Cl.: A61M 1/10

(54) **Pumpsystem, Schlauchpumpe und Schlauchkassette sowie Verfahren zum Konfigurieren eines Pumpsystems**
Pumping system, hose pump and hose cartridge and method for configuring a pumping system
Système de pompe, pompe tubulaire et cassette tubulaire ainsi que procédé de configuration d'un système de pompe

(30) Priorität: 26.06.2012 DE 102012012525
(43) Veröffentlichungstag der Anmeldung: 01.01.2014
(73) Patentinhaber: Karl Storz GmbH & Co. KG, 78532 Tuttlingen (DE); Storz Endoskop Produktions GmbH, 78532 Tuttlingen (DE)
(72) Erfinder: Emmerich, Bernd, 78576 Emmingen (DE); Puschke, Dirk, 78562 Tuttlingen (DE); Gümpel, Anika, 78531 Bodman-Ludwigshafen (DE); Burger, Matthias, 8207 Schaffhausen (CH); Schmidt, Manfred, 78476 Allensbach (DE); Stillhard, Otmar, 8266 Steckborn (CH)
(74) Vertreter: Fugmann, Winfried

(56) Entgegenhaltungen:
- US-A- 3 927 955
- US-A- 4 735 558
- US-A- 5 433 588
- US-A1- 2009 087 326
- US-A1- 2012 083 736

## Beschreibung

Die vorliegende Erfindung betrifft ein Pumpsystem, insbesondere zur medizinischen Verwendung, sowie eine Schlauchpumpe und eine Schlauchkassette zur Verwendung in einem derartigen Pumpsystem. Die Erfindung betrifft ferner ein Verfahren zum Konfigurieren eines Pumpsystems.

Bei chirurgischen Eingriffen besteht häufig die Notwendigkeit, Flüssigkeiten zu fördern, beispielsweise eine Spülflüssigkeit, die zum Spülen eines Operationsgebiets, zum Freihalten einer endoskopischen Optik oder zum Transport von zu entfernenden Gewebeteilen dient. Zum Fördern einer solchen Spülflüssigkeit zum Operationsgebiet und zum Absaugen der Spülflüssigkeit aus dem Operationsgebiet ebenso wie zum Absaugen von Körpersekreten sind Spül- und Saugpumpen bekannt, die in der Regel als Schlauchpumpen ausgebildet sind. Eine Schlauchpumpe weist mindestens einen Pumpenkopf auf, der eine Mehrzahl von Pumpelementen aufweist, die motorisch entlang einer geschlossenen Bahn geführt werden und dabei eine entlang eines Teilbereichs der Bahn angeordnete flexible Schlauchleitung fortschreitend zusammendrücken und dadurch in der Schlauchleitung enthaltene Flüssigkeit fördern. Durch die Klemmwirkung der Pumpelemente kann somit ein Überdruck zum Fördern von Flüssigkeit in ein Operationsgebiet erzeugt werden, ebenso wie durch die elastische Rückverformung des zusammengedrückten Schlauchs ein Unterdruck erzeugt werden kann, der zum Absaugen von Flüssigkeit aus dem Operationsgebiet genutzt werden kann. Schlauchpumpen haben den Vorteil, dass die Pumpe selbst und insbesondere der Pumpenkopf und die Pumpelemente nicht in direkten Kontakt mit dem geförderten Medium gelangen, so dass die Sterilitätsanforderungen, die insbesondere bei chirurgischen Anwendungen zu beachten sind, auf einfache Weise erfüllt werden können; die verwendeten Schläuche sind dabei für eine einmalige Verwendung ausgelegt. Schlauchpumpen, die auch als Schlauchquetschpumpen oder Peristaltikpumpen bezeichnet werden, kommen aber auch bei anderen medizinischen Anwendungen, etwa als Infusionspumpen oder in Dialysegeräten, sowie bei einer Vielzahl von nicht-medizinischen Anwendungen zum Einsatz.

Schlauchpumpen für medizinische Anwendungen sind üblicherweise als Rollenpumpen ausgebildet, bei denen die Pumpelemente auf dem Umfang einer motorisch drehbaren Scheibe angeordnet sind und um jeweils parallel zueinander stehende Achsen auf der Scheibe frei drehbar sind. Eine solche Rollenpumpe ist für die Ausübung einer Pumpwirkung und für die Steuerung der Fördermenge und des erzeugten Über- oder Unterdrucks besonders gut geeignet. Da die verwendeten Schläuche aus Sterilitätsgründen als Einwegteile ausgebildet sind und nach jedem Eingriff gewechselt werden müssen, ist es vor jeder Verwendung der Pumpe erforderlich, einen Schlauch an die Pumpelemente bzw. Pumprollen anzulegen, wobei auch auf den richtigen Anschluss der Zu- und Abführungsleitungen zu achten ist. Um den hiermit verbundenen Aufwand zu reduzieren, ist es bekannt, dass der Schlauch in einer Schlauchkassette aufgenommen ist, die mit der eigentlichen Pumpe gekoppelt werden kann und die eine Ausnehmung aufweist, in die die Pumprollen eingreifen, um zur Erzielung der Pumpwirkung auf den Schlauch einzuwirken.

Aus DE 199 60 668 C1 sowie aus DE 100 05 108 A1 ist eine Schlauchkassette für eine peristaltische Pumpe bekannt mit einem Kassettengehäuse und mit einem durch das Kassettengehäuse verlaufenden flexiblen Schlauch, der in dem Kassettengehäuse entlang eines Kreissegments geführt ist, wobei das Kassettengehäuse eine Ausnehmung zum Eingriff eines Rollenrades der peristaltischen Pumpe in das Innere des Kreissegments aufweist. Gemäß der hierbei gewählten Grundkonzeption wird der Schlauch durch eine Zugkraft geeigneter Größe mit genügendem Umfassungswinkel um das Rollenrad gezogen. Hierfür ist zumindest ein an das Kreissegment anschließender Schlauchschenkel in dem Kassettengehäuse in Längsrichtung des Schlauches zwischen einer Montageposition und einer Betriebsposition verschieblich gelagert. In einer ersten Montagehandlung wird die Kassette mit in Montageposition befindlichem Schlauchschenkel an ein Pumpengehäuse angesetzt, wobei zunächst Formschlussausnehmungen in einer Wandung der Kassette auf in diese eingreifende Haltestifte der Pumpe positioniert werden und danach durch Kippen der Kassette gegen die Außenwandung der Pumpe die Verbindung mit Kraftschlusselementen der Pumpe hergestellt wird. In einem weiteren Montageschritt wird der Schlauch durch Zug an dem verschieblichen Schlauchschenkel an das Rollenrad mit dem erforderlichen Druck angelegt.

Auch bei der in DE 10 2006 008 325 A1 offenbarten Schlauchkassette für eine peristaltische Pumpe wird von der Grundkonzeption ausgegangen, dass der Schlauch durch eine Zugkraft geeigneter Größe mit genügendem Umfassungswinkel um das Rollenrad der Pumpe gezogen wird. Die Schlauchkassette weist eine Ausnehmung für das Rollenrad auf, die sich entweder im Bereich einer Stirnfläche oder in einer Seitenfläche des Kassettengehäuses erstreckt. In beiden Fällen wird das Pumpschlauchsegment bei Bewegung der Schlauchkassette in die Betriebsposition um das Rollenrad gespannt.

Bei dem in DE 696 15 633 T2 beschriebenen Lade- und Entladesystem für chirurgische Kassetten wird der Schlauch durch das Zusammenwirken einer Kolbenkonstruktion mit einem vorragenden Abschnitt des Walzenkopfs einer peristaltischen Pumpe über den Walzenkopf gezogen. Gemäß DD 236 143 A1 ist der Schlauch zwischen zwei Elementen der Kassette eingebettet, wovon eines mit dem Gehäuse und das andere mit dem Rotor einer Pumpe verbindbar ist.

Aus US 4,798,580 ist ein mikrochirurgisches Spül-/Saugsystem bekannt, das ein Steuerungsgerät mit einem Rollenpumpenkopf und eine Schlauchkassette umfasst, die auf das Steuerungsgerät aufgesetzt werden kann, so dass der Rollenpumpenkopf ein in der Schlauchkassette befindliches Schlauchsegment gegen eine bogenförmige Wand drückt, um eine Pumpwirkung zu erzielen. Zum Ansetzen der Schlauchkassette an das Steuerungsgerät wird die Schlauchkassette in einem ersten Schritt mit zwei Laschen an dem dem Schlauchsegment entgegengesetzten Ende der Schlauchkassette hinter zwei Rückhalte klammem eingesetzt. In einem zweiten Schritt wird die Schlauchkassette an die hierfür vorgesehene Verbindungsfläche des Steuerungsgeräts herangeschwenkt. In einem dritten Schritt wird eine Nockenstange mit Hilfe eines Bügels gegen eine Seitenwand der Schlauchkassette geschwenkt, wodurch der in der Schlauchkassette aufgenommene Schlauchabschnitt zwischen dem Pumpenkopf und der Führungswand gequetscht wird.

In US 2009/0087326 A1 ist eine peristaltische Pumpenanordnung offenbart, die eine Einweg-Kassette und einen Pumpenkörper mit einem Haltestift umfasst. Der Pumpenkörper weist einen Kassettenaufnahmebereich auf, hinter den von oben die Kassette eingesetzt werden kann. Hierfür wird die Kassette zunächst mit einem Gelenklager auf den Haltestift des Pumpenkörpers aufgeschoben. Danach wird die Kassette auf dem Haltestift in die Installationsposition geschwenkt, in der ein Schlauch von den Rollen eines Rollenmechanismus gegen einen Laufring gedrückt wird, um eine Pumpwirkung zu erzielen. Dabei öffnet sich ein Rückhaltemittel des Pumpenkörpers und greift danach automatisch hinter ein Rückhaltemittel der Kassette, um diese in der Position zu halten.

US 5,433,588 offenbart eine peristaltische Pumpe mit einem Schlaucheinsatz und einem Deckelteil. Dabei wird zum Andrücken eines flexiblen Plastikschlauchs an Rollenelemente einer peristaltischen Pumpe zunächst mit einer Einwegeinheit ein Schlauchsegment horizontal an die Pumpe herangeführt. Danach wird das Deckelteil mit seitlichen Vorsprüngen in vertikale Schlitze eingesetzt und in diesen vertikal verschoben. Hierdurch wird der Schlauch zwischen den Rollenelementen eines Rotors und einer Oberfläche des Deckelteils zusammengedrückt, um Flüssigkeit durch den Schlauch zu fördern.

Gemäß US 4,735,558 umfasst eine Spül- und Saugeinrichtung eine Pumpenanordnung und eine Schlauchkassette. Die Schlauchkassette wird in das Pumpengehäuse eingeschoben, so dass die Rollen eines Pumpenkopfs einen flexiblen Schlauch gegen eine Wand drücken (s. D3, Fig. 1, 4; Spalte 3, Zeilen 40-59). Ein federbelasteter Rastmechanismus, der die Schlauchkassette mit dem Schlauch mit einer gewünschten Spannung an dem Pumpenkopf hält, umfasst bewegliche Rastmittel.

In US 2012/0083736 A1 ist eine Schlauchkassette offenbart, die eine Schlauchschleife aufweist, die um einen Rotor gespannt wird. Die Schlauchkassette rastet mit zwei Rastklauen in zwei Ausnehmungen am Pumpengehäuse ein. Ein Bereich einer Aufnahme der Kassette ist als schräge Haltewand ausgebildet, hinter die eine Schräge der Kassette greift, so dass eine Spannung im Schlauch die Kassette in der Aufnahme hält.

US 3,927,955 beschreibt eine medizinische Schlauchpumpe, bei der ein Schlauch in zwei gegeneinander schwenkbare Hälften einer Kassette eingelegt ist. Zum Andrücken des Schlauchs an die Rollen der Pumpe werden die Hälften geschwenkt und mit gefederten Fingern, die in Kerben der schwenkbaren Hälften eingreifen, gehalten.

Es ist Aufgabe der vorliegenden Erfindung, ein Pumpsystem mit einer Schlauchpumpe und einer Schlauchkassette sowie eine Schlauchpumpe und eine Schlauchkassette anzugeben, wobei die Kopplung der Schlauchkassette mit der Schlauchpumpe vereinfacht ist. Ferner ist es Aufgabe der vorliegenden Erfindung, ein Verfahren zum Konfigurieren eines Pumpsystems insbesondere für medizinische Anwendungen anzugeben, das einfacher ist und insbesondere eine erhöhte Sicherheit ermöglicht.

Diese Aufgabe wird durch ein Pumpsystem gemäß Anspruch 1, durch eine Schlauchpumpe gemäß Anspruch 13, durch eine Schlauchkassette gemäß Anspruch 14 sowie durch ein Verfahren gemäß Anspruch 15 gelöst.

Vorteilhafte Weiterbildungen der Erfindung ergeben sich aus den Unteransprüchen.

Ein erfindungsgemäßes Pumpsystem ist insbesondere für medizinische Anwendungen, vorzugsweise zur Verwendung bei einem chirurgischen Eingriff, ausgebildet. Das Pumpsystem umfasst eine Schlauchpumpe mit einer Gehäusewand und mit mindestens einem Pumpenkopf, der durch die Gehäusewand der Schlauchpumpe hindurch ragt. Im Rahmen dieser Anmeldung wird das Gerät, das den Pumpenkopf, einen Antrieb des Pumpenkopfs und ggf. Anzeige-, Bedienungs- und Steuerungseinrichtungen umfasst, als "Schlauchpumpe" bezeichnet. Die Schlauchpumpe kann ein weitgehend geschlossenes Gehäuse aufweisen, wobei die Gehäusewand, durch die der Pumpenkopf hindurchragt, das Gehäuse in der Betriebsposition der Schlauchpumpe in Richtung zu einem Bediener abschließt und Anzeige- und/oder Bedienelemente tragen kann. Der Pumpenkopf steht in Richtung zum Bediener aus der Gehäusewand hervor. Diese Richtung wird im Folgenden als "vorne" oder, im Zusammenhang mit der Schlauchkassette, auch als "oben" bezeichnet. Ein Teilbereich der Gehäusewand kann zum Erleichtern des richtigen Ansetzens einer Schlauchkassette besonders gekennzeichnet sein, etwa durch eine farbliche Markierung oder auch als erhabene oder vertiefte Fläche, die der Form der anzusetzenden Schlauchkassette in der dafür vorgesehenen Stellung entsprechen kann.

Der Pumpenkopf weist eine Mehrzahl an Pumpelementen auf, die durch einen Motor der Schlauchpumpe in einer geschlossenen Bahnkurve geführt werden. Die Pumpelemente können beispielsweise auf dem Umfang einer drehbaren Scheibe angeordnet sein und eine kreisförmige Bewegung um eine senkrecht zur Gehäusewand stehende Achse ausführen. Bei einer als Rollenpumpe ausgebildeten Schlauchpumpe sind die Pumpelemente frei drehbare Andruckrollen, die jeweils um ebenfalls senkrecht zur Gehäusewand stehende Achsen drehbar sind. Die Schlauchpumpe kann einen oder mehrere Pumpenköpfe aufweisen. Dementsprechend kann es vorgesehen sein, dass eine oder mehrere Schlauchkassetten gleichzeitig mit der Schlauchpumpe gekoppelt sein können.

Das Pumpsystem umfasst weiterhin mindestens eine mit der Schlauchpumpe durch Ansetzen an die Gehäusewand koppelbare Schlauchkassette. Die Schlauchkassette weist einen ersten Endbereich auf, in den ein Schlauchsegment einsetzbar ist. Ein eingesetztes Schlauchsegment ist in der Schlauchkassette derart gehalten, dass das Schlauchsegment in einer Andruckrichtung an mindestens ein Pumpelement des Pumpenkopfs andrückbar ist, so dass das Schlauchsegment mit dem Pumpenkopf zum Erzielen einer Pumpwirkung in dem Schlauchsegment zusammenwirken kann. Durch das Andrücken des Schlauchsegments an das mindestens eine Pumpelement des Pumpenkopfs wird eine Andruckrichtung definiert. Insbesondere wenn der Pumpenkopf als drehbare Scheibe ausgebildet ist und die Pumpelemente auf einer Kreisbahn mit zur Gehäusewand senkrechter Achse geführt werden, ist das Schlauchsegment in der Schlauchkassette vorzugsweise in Form eines Kreissegments mit beidseits tangential anschließenden Schenkeln geführt. Die Andruckrichtung, in der das Schlauchsegment an den Pumpenkopf bzw. an die Pumpelemente andrückbar ist, ist dann vom Scheitel des Kreissegments zum Krümmungsmittelpunkt des Kreissegments bzw. zur Drehachse der drehbaren Scheibe gerichtet. Die Schlauchkassette weist ferner einen zweiten Endbereich auf, der von dem ersten Endbereich in der Andruckrichtung beabstandet ist bzw. in der Andruckrichtung dem ersten Endbereich gegenüberliegend angeordnet ist.

Zum Erzielen einer Pumpwirkung in dem Schlauchsegment, d.h. zum Zusammenwirken mit dem Pumpenkopf, ist die Schlauchkassette an die Gehäusewand der Schlauchpumpe ansetzbar und wird dort derart gehalten, dass beim Betrieb der Schlauchpumpe die Pumpelemente das Schlauchsegment fortschreitend zusammendrücken und dadurch ein in dem Schlauchsegment enthaltenes flüssiges Medium transportieren.

Das Pumpsystem kann weiterhin ein Schlauchset umfassen, das zumindest das in die Schlauchkassette einsetzbare Schlauchsegment enthält. Zum Schlauchset können darüber hinaus insbesondere eine Zuführungsleitung und eine Abführungsleitung zum Zuführen bzw. Abfühlen des zu fördernden Mediums zum bzw. vom Schlauchsegment sowie Verbindungselemente zur Verbindung der Zu- und der Abführungsleitung mit dem Schlauchsegment gehören. Das Schlauchset und/oder die Schlauchkassette können als Einwegprodukt zur einmaligen Benutzung ausgebildet sein. Insbesondere kann die Schlauchkassette mit eingesetztem Schlauchsegment und ggf. an die Schlauchkassette angeschlossenen Zu- und Abführungsleitungen als zusammengebautes, steril verpacktes Einwegprodukt vorgesehen sein.

Bei einem erfindungsgemäßen Pumpsystem umfasst die Schlauchpumpe ein feststehendes, insbesondere von der Gehäusewand nach vorne ragendes oder in einem Abstand vor der Gehäusewand gehaltenes, erstes Halteelement zum formschlüssigen Halten des ersten Endbereichs der mindestens einen Schlauchkassette an der Gehäusewand. Das erste Halteelement kann beispielsweise als an bzw. vor der Gehäusewand angeordneter Haken oder Bügel ausgebildet sein. Die Schlauchkassette ist durch eine im Wesentlichen in Andruckrichtung verlaufende Bewegung mit dem ersten Halteelement verbindbar, beispielsweise indem die Schlauchkassette mit ihrem ersten Endbereich auf oder hinter das erste Halteelement geschoben werden kann. Das erfindungsgemäße Pumpsystem umfasst weiterhin ein feststehendes, insbesondere von der Gehäusewand nach vorne ragendes, zweites Halteelement zum Halten des zweiten Endbereichs der mindestens einen Schlauchkassette an der Gehäusewand, das beispielsweise als von der Gehäusewand nach vorne vorstehender Zapfen ausgebildet sein kann. Der zweite Endbereich kann insbesondere kraftschlüssig an der Gehäusewand gehalten werden. Erfindungsgemäß ist schließlich am zweiten Halteelement und/oder an der mindestens einen Schlauchkassette, insbesondere im oder nahe dem zweiten Endbereich der Schlauchkassette, eine Schräge angeordnet, durch die beim Ansetzen der Schlauchkassette an die Gehäusewand die Schlauchkassette in der Andruckrichtung verschoben wird. Die Schräge ist beispielsweise auf einer vom Pumpenkopf abgewandten Seite des zweiten Halteelements oder auf einer einem eingesetzten, kreissegmentartig geführten Schlauchsegment zugewandten Seite des zweiten Endbereichs angeordnet. Aufgrund der Schräge wirken somit das zweite Halteelement und die Schlauchkassette derart zusammen, dass beim Ansetzen der Schlauchkassette, etwa durch Ankippen oder Anschwenken, an die Gehäusewand, das Schlauchsegment an den Pumpenkopf bzw. an mindestens ein Pumpelement angedrückt wird.

Dadurch, dass die Schlauchkassette in einem ersten Endbereich, der ein eingesetztes Schlauchsegment umfasst, durch eine im Wesentlichen in Andruckrichtung verlaufende Bewegung mit dem ersten Halteelement verbindbar ist und dass das zweite Haltelement, das zum Halten des zweiten Endbereichs ausgebildet ist, durch eine Schräge mit der Schlauchkassette derart zusammenwirkt, dass das Schlauchsegment an den Pumpenkopf angedrückt wird, kann es erreicht werden, dass die Schlauchkassette in einer durchgehenden Bewegung und insbesondere in nur zwei Schritten mit der Schlauchpumpe koppelbar ist. In einem ersten Schritt wird der erste Endbereich mit dem ersten Halteelement verbunden, beispielsweise auf das erste Halteelement aufgeschoben oder hinter dieses geschoben, wobei bereits das Schlauchsegment mit dem mindestens einen Pumpelement in Anlage kommen kann. Dabei kann die Schlauchkassette in einer gegenüber der Gehäusewand nach vorne abgekippten Stellung gehalten werden. In einem zweiten Schritt wird in Fortführung der Bewegung des ersten Schritts die Schlauchkassette an die Gehäusewand angeschwenkt, wodurch nicht nur der zweite Endbereich der Schlauchkassette an der Gehäusewand gehalten werden kann, sondern gleichzeitig auch die zum Erzeugen der Pumpwirkung notwendige Andruckbewegung für ein Quetschen des Schlauchsegments durch die Pumpelemente ausgeführt wird. Hierdurch wird eine erhebliche Vereinfachung der zum Koppeln der Schlauchkassette mit der Schlauchpumpe notwendigen Handgriffe ermöglicht.

Gemäß einer bevorzugten Ausführungsform der Erfindung ist die Schlauchkassette als im Wesentlichen geschlossener, insbesondere U-förmiger Rahmen ausgebildet, der eine durchgehende Öffnung umschließt. Insbesondere kann die Schlauchkassette eine im Wesentlichen flache Unterseite und eine im Wesentlichen flache Oberseite aufweisen, wobei die Ober- und die Unterseite in der an die Gehäusewand angesetzten Position die Vorder- bzw. Rückseite der Schlauchkassette bilden. Der erste Endbereich der Schlauchkassette kann zum Halten des Schlauchsegments beispielsweise bogenförmig ausgebildet sein und eine Führungswand umfassen, an der das Schlauchsegment anliegen kann und gegen die es von den Pumpelementen gepresst werden kann. Der zweite Endbereich kann einen Basisbereich des U-förmigen Rahmens darstellen, in dem beispielsweise Schlauchanschlüsse oder weitere Funktionselemente der Schlauchkassette angeordnet sind.

Weiterhin ist es bevorzugt, dass das erste Halteelement der Schlauchpumpe als Haltezunge ausgebildet ist, die beim Ansetzen der Schlauchkassette an die Gehäusewand die Öffnung durchgreift und hinter die der erste Endbereich der Schlauchkassette zum formschlüssigen Halten an der Gehäusewand eingeschoben werden kann. Hierdurch wird die Schlauchkassette mit ihrem ersten Endbereich an der Gehäusewand gehalten. Dadurch, dass das erste Halteelement als Haltezunge ausgebildet ist, die eine durchgehende Öffnung der Schlauchkassette durchgreift, wird es ermöglicht, die Schlauchkassette in einer besonders kompakten Form auszubilden und auch die Halteelemente in einem eng begrenzten Verbindungsbereich auf der Gehäusewand anzuordnen, so dass ein großer Bereich der Gehäusewand für Bedien- und Anzeigeelemente freigehalten werden kann. Ferner kann es hierdurch auf einfache Weise ermöglicht werden, dass die Schlauchkassette bereits in einer gegenüber der Gehäusewand angekippten Position mit ihrem ersten Endbereich an der Gehäusewand gehalten werden kann. Schließlich kann es hierdurch beispielsweise auch verhindert werden, dass die Schlauchkassette zuerst mit ihrem zweiten und danach mit ihrem ersten Endbereich an die Gehäusewand angesetzt wird, da nach dem Ansetzen mit dem zweiten Endbereich der erste Endbereich der Schlauchkassette nicht mehr hinter die Haltezunge geschoben werden kann.

In vorteilhafter Weise ist die Haltezunge als eine nach vorne, d.h. in einer von der Gehäusewand weg weisenden Richtung, gekrümmte Führungszunge ausgebildet. Die Krümmung der Haltezunge nach vorne erleichtert das Einsetzen der Schlauchkassette weiter, indem der erste Endbereich der Schlauchkassette bei der Einsetzbewegung durch die Führungszunge in eine an der Verbindungsfläche gehaltene Position geführt wird.

Vorzugsweise ist das zweite Halteelement benachbart zum Pumpenkopf angeordnet und trägt die Haltezunge. Das zweite Halteelement ist insbesondere in der Andruckrichtung, die entsprechend dem Ansetzen der Schlauchkassette zum Andrücken des Schlauchsegments an den Pumpenkopf auch relativ zur Gehäusewand definiert ist, gegenüber dem Pumpenkopf versetzt und steht ebenfalls aus der Gehäusewand vor. Das zweite Halteelement kann beispielsweise als Haltezapfen oder Haltesockel ausgebildet sein, der beim Ansetzen der Schlauchkassette an die Gehäusewand in die Öffnung der Schlauchkassette eingreift oder diese durchgreift und an dessen Vorderseite die Haltezunge angeordnet ist. Die Haltezunge kann beispielsweise derart angeordnet sein, dass sie über den Pumpenkopf greift oder über diesen hinaus greift. Hierdurch wird eine besonders kompakte Anordnung der zum Ankoppeln der Schlauchkassette an die Schlauchpumpe notwendigen Bauelemente an der Gehäusewand ermöglicht.

Weiterhin ist es bevorzugt, dass eine mit dem zweiten Halteelement zusammenwirkende Innenseite der Öffnung der Schlauchkassette die Schräge zur Bewirkung der Verschiebung in Andruckrichtung aufweist und dass das zweite Halteelement eine beim Ansetzen der Schlauchkassette, insbesondere beim Anschwenken mit dem zweiten Endbereich an die Gehäusewand, auf die Schräge wirkende gefederte Raste aufweist. Die gefederte Raste kann beispielsweise als Kugelraste ausgebildet sein. Hierdurch kann auf einfache und sichere Weise die Verschiebung in Andruckrichtung ermöglicht werden. Ferner kann erreicht werden, dass die Schlauchkassette mit ihrem zweiten Endbereich mit vorgebbarer Kraft kraftschlüssig an der Schlauchpumpe gehalten und beim Ansetzen die zum Erzielen der Pumpwirkung notwendige Quetschkraft aufgebracht wird.

Gemäß einer besonders bevorzugten Ausführungsform der Erfindung weist die Innenseite der Schlauchkassette, die die Schräge trägt und die mit dem zweiten Halteelement zusammenwirkt, eine Rastvertiefung auf, in die die gefederte Raste eingreifen und dadurch die Schlauchkassette in der angekoppelten Position an der Gehäusewand halten kann. Hierdurch kann auf einfache Weise erreicht werden, dass die Schlauchkassette sicher an der Gehäusewand gehalten ist und mit einer vorbestimmbaren Kraft von der Schlauchpumpe wieder getrennt werden kann.

In vorteilhafter Weise sind die Federkraft und der Federweg der gefederten Raste derart bemessen, dass auch nach Ansetzen an die Gehäusewand zunächst eine geringfügige Verschiebung der Schlauchkassette in oder entgegen der Andruckrichtung möglich ist, um Toleranzen hinsichtlich des Durchmessers und/oder der Komprimierbarkeit des gegen den Pumpenkopf gepressten Schlauchsegments auszugleichen. Hierfür ist insbesondere die Federkraft so gewählt, dass die vom Schlauchsegment ausgeübte elastische Rückstellkraft zumindest näherungsweise kompensiert wird. Das erste und/oder das zweite Halteelement können ferner zur Führung der Schlauchkassette bei einer derartigen Ausgleichsbewegung ausgebildet sein. Wenn die Schlauchkassette bezüglich einer zu einer Ober- und Unterseite senkrechten Mittelebene näherungsweise symmetrisch ausgebildet ist, befindet sich die Rastvertiefung vorzugsweise innerhalb der Mittelebene. Hierdurch wird auf einfache Weise eine weiter verbesserte Befestigung sowie eine verbesserte Funktion der Schlauchkassette an der Schlauchpumpe ermöglicht.

Gemäß einer besonders bevorzugten Ausführungsform der Erfindung ist die Schräge als bogenförmig konvex gekrümmte Rampe ausgebildet. Hierdurch wird es ermöglicht, dass die mit zunehmender Längsverschiebung der Schlauchkassette beim Anschwenken des zweiten Endbereichs an die Gehäusewand entsprechend der elastischen Verformbarkeit des gequetschten Schlauchsegments zunehmende Gegenkraft überwunden werden kann, ohne dass die zum Anschwenken notwendige Kraft erheblich zunimmt. Hierdurch wird das Verbinden der Schlauchkassette mit der Schlauchpumpe weiter erleichtert.

Weiterhin ist es bevorzugt, dass an der Gehäusewand ein um eine näherungsweise senkrecht zur Gehäusewand stehende Achse schwenkbarer Verriegelungshebel angeordnet ist. In vorteilhafter Weise kann das zweite Halteelement den Verriegelungshebel tragen, insbesondere kann der Verriegelungshebel auf der vom zweiten Halteelement getragenen Haltezunge bzw. an dem von der Gehäusewand abstehenden Ende eines als Haltesockel oder Haltezapfen ausgebildeten zweiten Halteelements angeordnet sein. Alternativ kann der Verriegelungshebel auch an einer anderen Position an der Gehäusewand, beispielsweise ober- oder unterhalb des zum Ansetzen der Schlauchkassette bestimmten Bereichs, angeordnet sein. Der Verriegelungshebel ist zum Übergreifen und formschlüssigen Halten der Schlauchkassette an der Gehäusewand schwenkbar. Nach dem Anschwenken des zweiten Endbereichs an die Gehäusewand und ggf. nach dem Einrasten der gefederten Raste kann durch Drehen des Verriegelungshebels eine zusätzliche Sicherung gegen ein unbeabsichtigtes Lösen der Schlauchkassette erreicht werden.

In besonders vorteilhafter Weise weist die Schlauchkassette auf ihrer Oberseite, die in der an die Gehäusewand angesetzten Lage die Vorderseite ist, eine entlang einer Bahn des Endbereichs des Verriegelungshebels verlaufende ansteigende Rampe auf. Im Endbereich des Verriegelungshebels kann auf dessen Rückseite ein insbesondere federnd gelagertes Druckstück angeordnet sein. Beim Schwenken des Verriegelungshebels zum Sichern der Schlauchkassette an der Gehäusewand wird es hierdurch ermöglicht, dass entsprechend der ansteigenden Ausbildung der Rampe eine mit zunehmendem Schwenkwinkel zunehmende, ggf. durch die federnde Lagerung des Druckstücks begrenzte, Kraft auf die Schlauchkassette ausgeübt wird. Hierdurch ist eine besonders sichere, feste und reproduzierbare Befestigung der Schlauchkassette an der Schlauchpumpe erreichbar.

Vorzugsweise weist die Rampe in einem Endbereich eine Rastfläche auf. Diese kann etwa als durch eine Stufe abgesetzte Endfläche ausgebildet sein, worauf der Verriegelungshebel bzw. das Druckstück nach Erreichen eines vorgegebenen maximalen Schwenkwinkels einrastet. Hierdurch wird die Bedienung des Pumpsystems weiter vereinfacht, indem hierdurch das Erreichen des vorgegebenen Schwenkwinkels besonders einfach erkennbar ist. Ferner wird eine zusätzliche Sicherheit gegen unbeabsichtigtes Lösen des Verriegelungshebels erreicht.

Gemäß einer besonders bevorzugten Ausführungsform der Erfindung weist die Schlauchkassette mindestens eine Druckmessmembran auf, die in Verbindung mit einem durch das Schlauchsegment geförderten Fluid steht. Die Druckmessmembran ist zum Zusammenwirken mit einem Drucksensor der Schlauchpumpe angeordnet und gelangt beim Koppeln der Schlauchkassette mit der Schlauchpumpe in Kontakt mit einer Messfläche des Drucksensors der Schlauchpumpe. Die Druckmessmembran, der Verriegelungshebel und die Rampe sind hierbei vorzugsweise derart angeordnet, dass der Verriegelungshebel in einem Endbereich der ansteigenden Rampe bzw. beim Erreichen der Rastfläche die Schlauchkassette im Bereich der Druckmessmembran an die Gehäusewand und damit die Druckmessmembran an die Messfläche des Drucksensors andrückt. Hierdurch wird eine einfache, sichere und weitgehend reproduzierbare und somit fehlerfreie bzw. rauscharme Druckmessung ermöglicht, die eine genaue Steuerung der Pumpenfunktion ermöglicht.

Eine erfindungsgemäße Schlauchpumpe weist mindestens einen durch eine Gehäusewand der Schlauchpumpe hindurch ragenden Schlauchpumpenkopf auf sowie ein feststehendes erstes Halteelement, mit dem mindestens eine Schlauchkassette durch eine im Wesentlichen in einer Andruckrichtung verlaufende Bewegung verbindbar ist, zum formschlüssigen Halten eines ersten Endbereichs der Schlauchkassette an der Gehäusewand und ein feststehendes zweites Halteelement zum Halten eines zweiten Endbereichs der mindestens einen Schlauchkassette an der Gehäusewand, wobei die Schlauchkassette in einer kontinuierlichen Bewegung in Andruckrichtung mit der Schlauchpumpe verbindbar ist. Die erfindungsgemäße Schlauchpumpe ist insbesondere als Teil des oben beschriebenen Pumpsystems ausgebildet.

Eine erfindungsgemäße Schlauchkassette ist derart ausgebildet, dass sie durch Ansetzen an die Gehäusewand einer Schlauchpumpe mit dieser koppelbar ist, und umfasst einen ersten Endbereich, in dem ein Schlauchsegment zum Andrücken in einer Andruckrichtung an mindestens ein Pumpelement des Pumpenkopfs haltbar ist, und einen zweiten, vom ersten in der Andruckrichtung beabstandeten Endbereich, wobei der erste Endbereich der Schlauchkassette an der Gehäusewand durch ein erste Halteelement der Schlauchpumpe formschlüssig an der Gehäusewand haltbar ist und der zweite Endbereich der Schlauchkassette durch ein zweites Halteelement der Schlauchpumpe an der Gehäusewand insbesondere kraftschlüssig haltbar ist, wobei die Schlauchkassette in einer kontinuierlichen Bewegung in Andruckrichtung mit der Schlauchpumpe verbindbar ist. Die erfindungsgemäße Schlauchkassette ist insbesondere als Teil des oben beschriebenen Pumpsystems ausgebildet. Die Schlauchkassette ist vorzugsweise zur einmaligen Benutzung bestimmt.

Bei einem erfindungsgemäßen Verfahren zum Zusammenstellen bzw. Konfigurieren eines insbesondere medizinischen Schlauchpumpensystems werden eine Schlauchpumpe mit einem Gehäuse, das eine Gehäusewand aufweist, durch die ein Pumpenkopf hinausragt, eine mit der Schlauchpumpe zum Zusammenwirken zum Fördern eines Pumpmediums koppelbare Schlauchkassette und ein Schlauchset bereitgestellt. Die Gehäusewand weist insbesondere eine Verbindungsfläche auf, an die die Schlauchkassette angesetzt wird. Das Schlauchset umfasst einen Zuleitungsschlauch und einen Ableitungsschlauch für das Pumpmedium, ein in die Schlauchkassette einsetzbares Schlauchsegment und zwei Verbindungselemente, wobei die Verbindungselemente entsprechend der vorgesehenen Verwendung bei einem chirurgischen Eingriff ausgewählt sind und ggf. eine entsprechende Druckmessmembran und/oder eine entsprechende Kodierung aufweisen. Der Zuleitungsschlauch und der Ableitungsschlauch werden über die Verbindungselemente mit dem Schlauchsegment verbunden. Sodann werden das Schlauchsegment und die Verbindungselemente in die Schlauchkassette eingesetzt, wobei das Schlauchsegment in einen ersten Endbereich eingesetzt wird und die Verbindungselemente in den zweiten Endbereich. Die Schlauchkassette kann auch in vormontierter Form mit bereits eingesetztem Schlauchsegment sowie ggf. mit angeschlossenen Zu- und Ableitungsschläuchen und mit den Verbindungselementen, die eine entsprechende Druckmessmembran und/oder eine entsprechende Kodierung aufweisen können, bereitgestellt werden.

Die Schlauchkassette wird nun in abgekippter Lage an die Gehäusewand, insbesondere an die Verbindungsfläche, angesetzt, so dass der erste Endbereich der Schlauchkassette in Berührung mit dem Gehäuse der Schlauchpumpe steht, während der zweite Endbereich von diesem noch abgehoben ist. Der erste Endbereich wird sodann in einer Andruckrichtung in ein erstes Halteelement zur formschlüssigen Verbindung des ersten Endbereichs mit der Schlauchpumpe bis zur Anlage des in der Schlauchkassette eingesetzten Schlauchsegments an mindestens einem Pumpelement des Pumpenkopfs eingeschoben. Danach wird die Schlauchkassette mit ihrem zweiten Endbereich an die Gehäusewand bzw. die Verbindungsfläche herangeschwenkt, wobei die Schlauchkassette über eine Schräge eines zweiten Halteelements und/oder der Schlauchkassette automatisch in der Andruckrichtung verschoben und dadurch das Schlauchsegment an das mindestens eine Pumpelement angedrückt wird, insbesondere soweit angedrückt wird, dass das Schlauchsegment zum Erzielen der Pumpwirkung abgequetscht wird. In der herangeschwenkten Lage wird die Schlauchkassette durch das erste und das zweite Halteelement an der Gehäusewand gehalten, insbesondere durch das erste Halteelement formschlüssig und durch das zweite Halteelement kraftschlüssig, etwa durch Reibung zwischen der Schlauchkassette und dem zweiten Halteelement oder durch eine gefederte Raste. Schließlich kann die Schlauchkassette durch Drehen eines Verriegelungshebels, der auf die Vorderseite der Schlauchkassette drückt, in dieser Position, in der die Schlauchkassette zum Zusammenwirken mit der Schlauchpumpe zum Fördern des Pumpmediums zusammenwirkt, verriegelt werden. Zum Betrieb des Pumpsystems werden der Zu- und der Ableitungsschlauch an medizinische, insbesondere chirurgische Instrumente bzw. an einen Versorgungs- oder einen Entsorgungsbehälter angeschlossen; dies kann auch in einem früheren Schritt geschehen sein.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1a und 1b eine Schlauchpumpe gemäß einem Ausführungsbeispiel der Erfindung in zwei Ansichten;
Fig. 2a und 2b eine Schlauchkassette mit eingesetzten Verbindungselementen in zwei Ansichten sowie Fig. 2c ein Detail einer Variante der in Fig. 2a und 2b dargestellten Schlauchkassette;
Fig. 3a und 3b eine Schlauchkassette mit eingesetzten Schläuchen in zwei Ansichten;
Fig. 4a und 4b einen Zwischenzustand beim Ansetzen einer Schlauchkassette an eine Schlauchpumpe sowie eine Schlauchpumpe mit angesetzter Schlauchkassette;
Fig. 5a bis 5c in schematischer Form verschiedene Konfigurationen zur Verwendung des Pumpsystems.

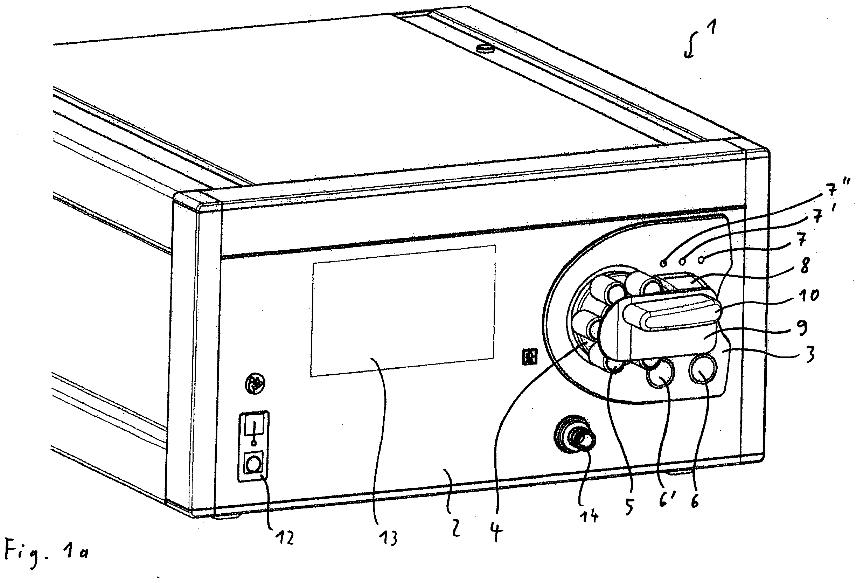

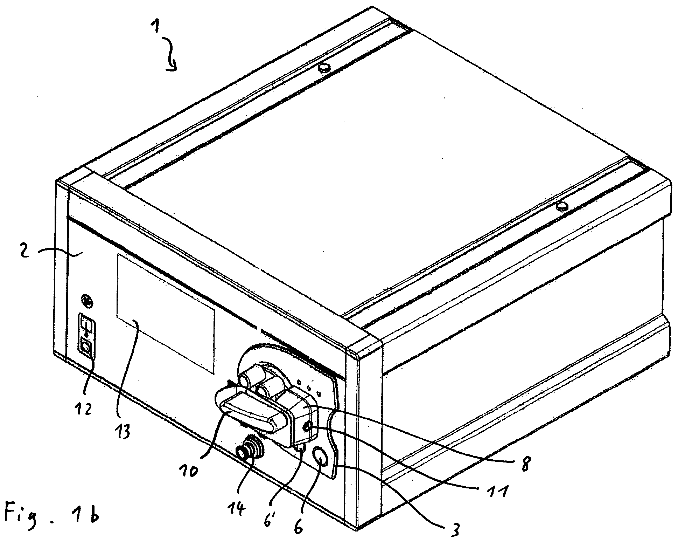

In den Figuren 1a und 1b ist eine Schlauchpumpe, die als Rollenpumpe 1 ausgebildet ist, gemäß einem Ausführungsbeispiel der Erfindung gezeigt. Die Rollenpumpe 1 weist ein weitgehend geschlossenes Gehäuse mit einer vorderen Gehäusewand 2 auf. Eine Verbindungsfläche 3 zum Ansetzen einer Schlauchkassette ist farblich sowie durch eine in Form und Größe der Rückwand der Schlauchkassette entsprechende Erhöhung auf der Gehäusewand 2 der Rollenpumpe 1 gekennzeichnet; hierdurch wird für einen Benutzer das lagerichtige Ansetzen der Schlauchkassette erleichtert. In oder vor der Verbindungsfläche 3 befindet sich eine durch einen in der Rollenpumpe 1 aufgenommenen Motor um eine senkrecht zur Verbindungsfläche 3 stehende Achse drehbare Scheibe 4, an deren Umfang eine Mehrzahl an Rollen 5 angeordnet sind, die um ebenfalls senkrecht zur Verbindungsfläche 3 stehende Achsen an der Scheibe 4 frei drehbar gelagert sind. Die Verbindungsfläche 3 weist einen Drucksensor 6 auf, der beispielsweise als Membran mit einem dahinter angeordneten Messwertgeber ausgebildet sein kann. Zur alternativen Verwendung oder auch zur gleichzeitigen Verwendung zur Erhöhung der Genauigkeit ist ein weiterer Drucksensor 6' ggf. mit einer weiteren Membran vorgesehen. Weiterhin weist die Rollenpumpe eine Mehrzahl an Reflektionslichtschranken 7, 7', 7" auf, die zum Zusammenwirken mit entsprechenden Reflektionsflächen eines Verbindungselements angeordnet sind (siehe unten). Benachbart zum Pumpenkopf, der durch die Scheibe 4 und die Rollen 5 gebildet wird, steht aus der Verbindungsfläche 3 ein Haltesockel 8 hervor, der an seinem vorderen Ende ein als Zunge 9 ausgebildetes Halteelement sowie einen um eine senkrecht zur Verbindungsfläche 3 stehende Achse nach unten schwenkbaren Verriegelungshebel 10 trägt. Die Zunge 9 ist an ihrem über den Pumpenkopf greifenden Ende nach vorne gewölbt. In Fig. 1b ist an der von dem Pumpenkopf abgewandten Rückseite des Haltesockels 8 eine gefedert gelagerte Rastkugel 11 erkennbar.

Die Rollenpumpe 1 weist einen Netzschalter 12 und ein als Touchscreen ausgebildetes Bedienfeld 13 zum Anzeigen und Verändern von Betriebsparametern sowie ggf. zum Anzeigen weiterer Daten auf. Die Rollenpumpe 1 kann an der vorderen Gehäusewand 2 oder auch an der hinteren Gehäusewand weitere Anschlüsse beispielsweise für elektrische Leitungen aufweisen, wobei in Fig. 1a und 1b exemplarisch ein Anschluss 14 dargestellt ist. Die Rollenpumpe 1 kann im Rahmen eines integrierten Steuerungssystems des Operationssaals betreibbar sein und beispielsweise über eine zentrale Steuerung, die eine Sprachsteuerung umfassen kann, angesteuert werden.

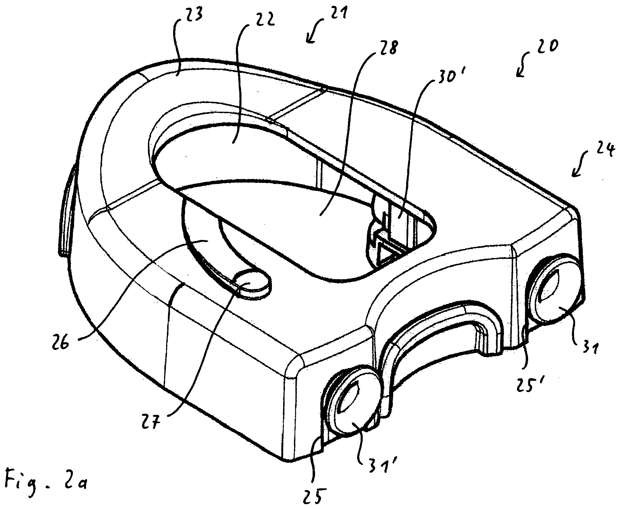

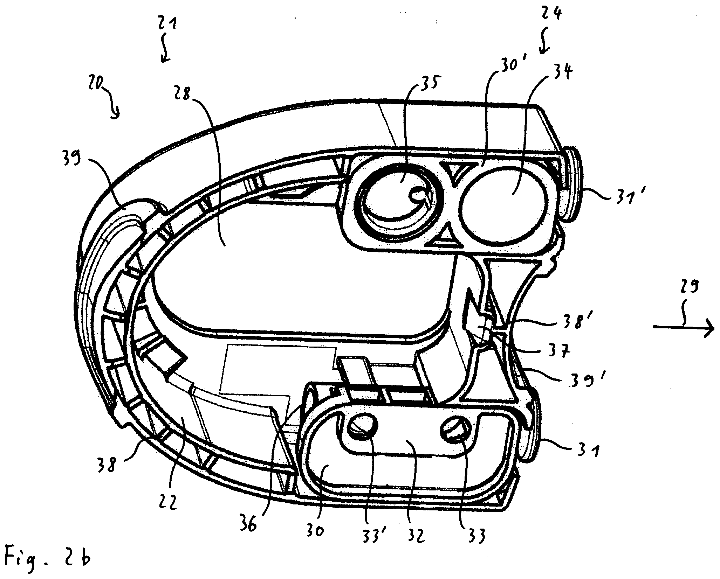

In Fig. 2a und 2b ist eine Schlauchkassette 20, die mit der Rollenpumpe 1 verbindbar ist, schematisch in zwei verschiedenen Ansichten gezeigt. Wie in Fig. 2a dargestellt, ist die Schlauchkassette 20 als geschlossener U-förmiger Rahmen mit einer im Wesentlichen flachen Oberseite ausgebildet. Ein erster Endbereich 21 der Schlauchkassette 20, der bogenförmig ausgebildet ist, umfasst eine in der Darstellung der Fig. 2a teilweise verdeckte bogenförmige Führungswand 22, an der ein Schlauchsegment in Anlage haltbar ist. Auf der Oberseite der Schlauchkassette ist im ersten Endbereich 21 eine Erhöhung 23 angeordnet. In einem zweiten Endbereich 24, der einen Basisbereich des U-förmigen Rahmens darstellt, sind Ausbrüche 25, 25' zum Durchführen einer Zu- und einer Abführungsleitung vorgesehen. Die Oberseite der Schlauchkassette weist in ihrem mittleren Bereich eine ansteigende, kreissegmentförmige Rampe 26 auf, die in ihrem Endbereich eine nach unten leicht abgestufte Endfläche 27 aufweist. Der U-förmige Rahmen umschließt eine durchgehende Öffnung 28. In die Schlauchkassette sind zwei Verbindungselemente eingesetzt, wovon in Fig. 2a das Verbindungselement 30' sowie die Schlauchanschlüsse 31, 31' erkennbar sind.

In Fig. 2b ist die Schlauchkassette 20 von ihrer Unterseite gezeigt, wobei die in die Schlauchkassette 20 eingesetzten Verbindungselemente 30, 30' sichtbar sind. Diese sind von der Schlauchkassette 20 trennbar und werden zum Verbinden mit einem Schlauchsegment sowie dem Zu- und dem Ableitungsschlauch aus der Schlauchkassette 20 entnommen oder werden separat bereitgestellt. Nach Verbinden mit den Schläuchen werden die Verbindungselemente 30, 30' in die Schlauchkassette 20 eingesetzt. Der Übersichtlichkeit halber sind in Fig. 2a und 2b die Schläuche nicht dargestellt.

Das erste Verbindungselement 30 weist eine Reflektionsfläche 32 auf, die beim Ansetzen der Schlauchkassette 20 an die Verbindungsfläche 3 der Rollenpumpe 1 in der Betriebsposition den Reflektionslichtschranken 7, 7', 7" gegenüberliegend angeordnet ist und mit diesen zusammenwirkt. Die Reflektionsfläche 32 kann beispielsweise zwei Ausnehmungen 33, 33' aufweisen und ist in einem dazwischenliegenden Bereich durchgehend ausgeführt, so dass nur die mittlere Reflektionslichtschranke 7' ein Reflektionssignal liefert. Die Anzahl und Anordnung der Ausnehmungen ermöglicht eine Kodierung der Schlauchkassette, so dass durch eine Steuerungseinrichtung der Rollenpumpe 1 automatisch Betriebsparameter eingestellt werden können, die einer dadurch kodierten Verwendung entsprechen, oder bei einer nicht geeigneten Konfiguration ggf. ein Warnsignal abgegeben oder die Rollenpumpe nicht betrieben werden kann.

Das zweite Verbindungselement 30' weist eine Druckmessmembran 34 auf sowie eine Ausnehmung 35 für eine weitere Druckmessmembran. Die Druckmessmembran steht auf ihrer Innenseite mit einem Innenraum des Verbindungselements 30' in Verbindung, der beim Betrieb des Pumpsystems mit dem zu fördernden Pumpmedium gefüllt ist, und ist zum Zusammenwirken mit dem Drucksensor 6 der Rollenpumpe 1 angeordnet (s. Fig. 1a, 1b). Alternativ oder zusätzlich kann eine weitere Druckmessmembran in der Ausnehmung 35 angebracht werden; fehlt diese, ist die Ausnehmung 35 fluiddicht verschlossen.

An die Verbindungselemente 30, 30' können über die Schlauchanschlüsse 31, 31' ein Zu- bzw. ein Ableitungsschlauch angeschlossen werden sowie über jeweils einen weiteren Schlauchanschluss, von denen in Fig. 2b der Schlauchanschluss 36 des Verbindungselements 30 zu erkennen ist, das Schlauchsegment, das hierdurch an der Führungswand 22 gehalten wird. In Fig. 2b ist ferner eine an der Innenwand der Öffnung 28 gegenüber der Führungswand 22 angeordnete, zur Führungswand 22 gerichtete Schräge 37 gezeigt, über die beim Anschwenken der Schlauchkassette 20 an die Verbindungsfläche 3 die Rastkugel 11 läuft (s. Fig. 1b). Die Schräge ist derart geneigt, dass sie von der Unterseite der Schlauchkassette 20 ausgehend zur Oberseite hin einen abnehmenden Abstand von der Führungswand 22 hat. Hierdurch wird beim Anschwenken der Schlauchkassette 20 an die Verbindungsfläche 3 eine Verschiebung der Schlauchkassette 20 in einer durch den Pfeil 29 angedeuteten Andruckrichtung und dadurch eine in der Andruckrichtung wirkende Andruckkraft zum Andrücken des an der Führungswand 22 anliegenden Schlauchsegments an eine oder mehrere Rollen 5 erzeugt. Oberhalb der Schräge 37 kann die Innenwand der Öffnung 28 eine Vertiefung zur rastenden Aufnahme der Rastkugel 11 aufweisen (nicht dargestellt). Zur Verstärkung der Führungswand 22 und des Basisbereichs der Schlauchkassette 20, der die Schräge 37 trägt, können Verbindungsrippen 38, 38' sowie Verstärkungswülste 39, 39' vorgesehen sein.

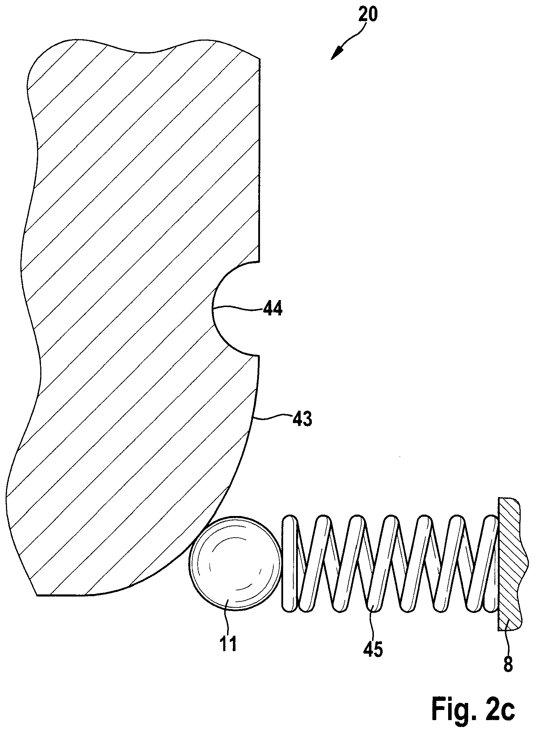

In einer Variante der Schlauchkassette 20, die im Übrigen wie in Fig. 2a und 2b ausgebildet ist, kann die Innenwand der Öffnung 28 anstelle einer zumindest abschnittsweise ebenen Schräge 37 eine in Fig. 2c schematisch im Querschnitt dargestellte, bogenförmig konvex gewölbte Rampe 43 aufweisen, über die die am Haltesockel 8 gelagerte Rastkugel 11 beim Anschwenken der Schlauchkassette 20 an die Verbindungsfläche 3 läuft (s. Fig. 1b). Die Rampe 43 ist ebenfalls derart geneigt, dass beim Anschwenken der Schlauchkassette 20 eine Verschiebung der Schlauchkassette 20 in Andruckrichtung und dadurch eine entsprechende Andruckkraft erzeugt wird. Oberhalb der Rampe 43 ist eine Vertiefung 44 zur rastenden Aufnahme der Rastkugel 11 angeordnet, in die die Rastkugel 11 zum Halten der Schlauchkassette 20 an der Verbindungsfläche 3 unter der Kraft der in Fig. 2c symbolisch dargestellten Feder 45 einrastet. Die Spannung der Feder 45 nach Überwinden der Schräge 37 bzw. der Rampe 43 ist so bemessen, dass auch dann, wenn die Kugel 11 in die Vertiefung 44 eingerastet ist, eine geringe Bewegung der Schlauchkassette 20 an der Verbindungsfläche 3 zum Ausgleich von Toleranzen der Dicke und Festigkeit eines in die Schlauchkassette 20 eingesetzten Schlauchsegments möglich ist.

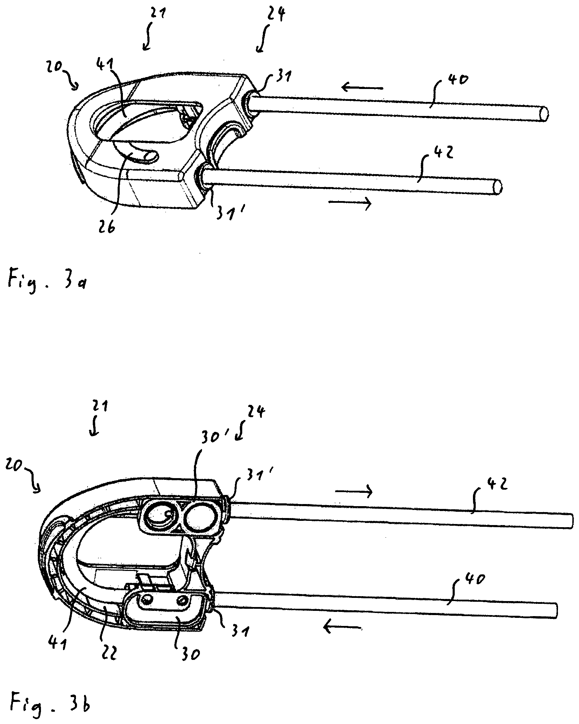

In der Darstellung der Fig. 3a und 3b ist die Schlauchkassette 20 mit montierten Schläuchen gezeigt. Der Zuleitungsschlauch 40 ist über das Verbindungselement 30 mit dem Schlauchsegment 41 verbunden, welches wiederum über das Verbindungselement 30' mit dem Ableitungsschlauch 42 verbunden ist. Die Verbindungselemente 30, 30' sind in entsprechende Ausnehmungen der Schlauchkassette eingesetzt, wodurch das Schlauchsegment 41 an der bogenförmigen Führungswand 22 anliegt oder nahezu anliegt. Der Zuleitungs- und der Ableitungsschlauch 40, 42 sind an der Basis des U-förmigen Rahmens zur Schlauchkassette 20 geführt bzw. von dieser weggeführt. Die durch die Drehrichtung der Scheibe 4 der Rollenpumpe vorgegebene Flussrichtung eines geförderten Pumpmediums, beispielsweise einer Spülflüssigkeit, ist in Fig. 3a und 3b durch die Pfeile angedeutet.

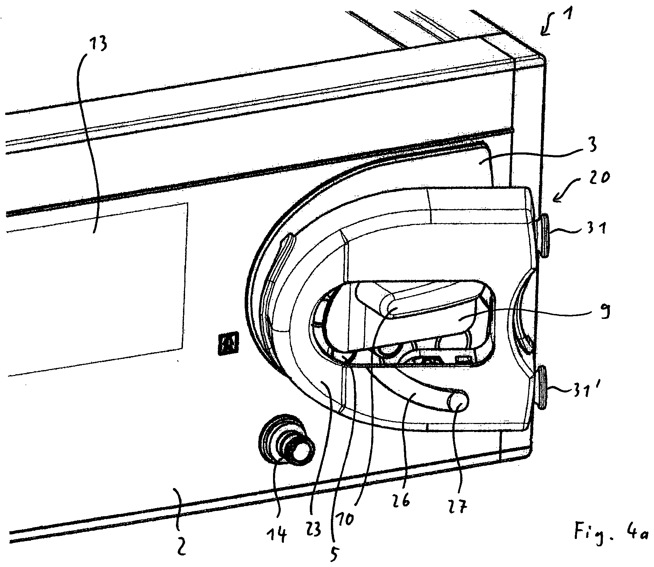

In Fig. 4a ist das Ansetzen der Schlauchkassette 20 an die vordere Gehäusewand 2 der Rollenpumpe 1 bzw. an die Verbindungsfläche 3 dargestellt, wobei der Zu- und der Ableitungsschlauch 40, 42 der Übersichtlichkeit halber nicht gezeigt sind. Die Unterseite der Schlauchkassette 20 ist dabei nach hinten, d.h. zur Verbindungsfläche 3, gerichtet, während die Oberseite der Schlauchkassette 20 zu einem Bediener nach vorne gerichtet ist. Wie in Fig. 4a gezeigt, wird die Schlauchkassette 20 zunächst in einer von der Verbindungsfläche 3 um eine vertikale Achse gekippten Stellung mit ihrem ersten Endbereich 21 an die Verbindungsfläche 3 angesetzt. Die Zunge 9 greift dabei durch die Öffnung der Schlauchkassette hindurch. Die Schlauchkassette 20 wird dann hinter die Zunge 9 geschoben, wobei die nach vorne gewölbte Ausbildung des Endbereichs der Zunge 9 das Einführen der Schlauchkassette 20 erleichtert. Die Schlauchkassette 20 wird gemäß der Darstellung der Fig. 4a so weit unter die Zunge 9 geschoben, bis ein an der Führungswand 22 anliegendes Schlauchsegment 41 (s. Fig. 3a, 3b) an den Rollen 5 des Pumpenkopfs, die in die Öffnung der Schlauchkassette 20 eingreifen, anliegt.

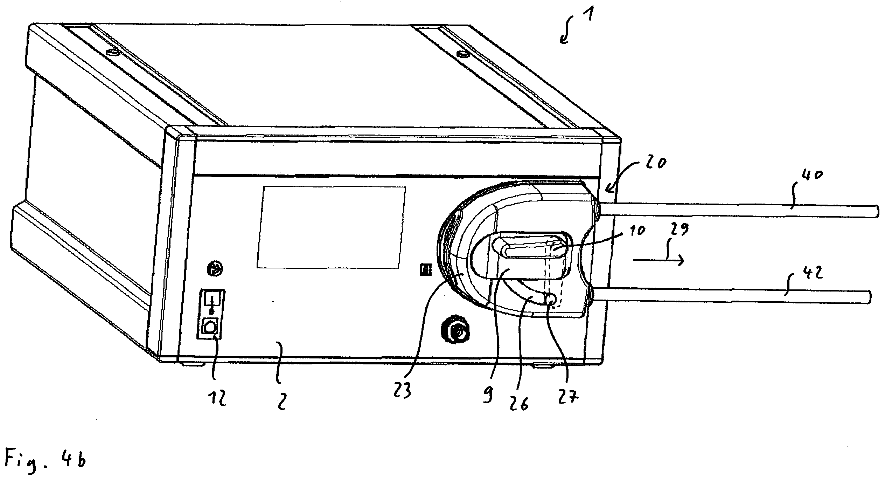

Im nächsten Schritt wird die Schlauchkassette 20 mit ihrem zweiten Endbereich 24 nach hinten geschwenkt und in Anlage mit der Verbindungsfläche 3 gebracht; diese Position ist in Fig. 4b gezeigt. Über die Schräge 37 an der Innenseite der Öffnung 28 der Schlauchkassette, die mit der Rastkugel 11 an der vom Pumpenkopf entfernten Rückseite des Haltesockels 8 zusammenwirkt, wird die Schlauchkassette in der durch den Pfeil 29 angedeuteten Andruckrichtung verschoben, so dass das Schlauchsegment 41 an die Rollen 5 angedrückt und zwischen der Führungswand 22 und den Rollen 5 gequetscht wird. Dabei wird das Maß der Verschiebung auch durch die Federung der Rastkugel bestimmt, so dass mögliche Toleranzen hinsichtlich Durchmesser und Komprimierbarkeit des Schlauchsegments 41 kompensiert werden können. In einer maximal verschobenen Position kann die Erhöhung 23 der Schlauchkassette 20 in Anlage mit der Rückseite der Vorwölbung der Zunge 9 gelangen. Die Druckmessmembran 34 liegt in Übereinstimmung mit dem Drucksensor 6 der Rollenpumpe 1, und die Reflektionsfläche 36 kommt gegenüber den Reflektionslichtschranken 7, 7', 7" zu liegen. Die Druckmessmembran 34 und die Reflektionsfläche 32 sind so bemessen, dass sowohl die Druckmessung als auch die Funktion der Reflektionslichtschranken 7, 7', 7" durch die zum Toleranzausgleich notwendige geringfügige Verschiebung der Schlauchkassette 20 nicht beeinträchtigt werden.

Die Schlauchkassette 20 kann somit insbesondere mit einer Hand gegriffen und durch eine durchgehende Bewegung mit der Schlauchpumpe 1 gekoppelt werden, wobei außerdem ein Andrücken des Schlauchsegments an den Pumpenkopf erfolgt und eine Druckmessung der geförderten Flüssigkeit ermöglicht wird. Ferner wird durch die Zunge 9 und die Erhöhung 23 ein Koppeln der Schlauchkassette in nur einer Ausrichtung ermöglicht, wodurch ein fehlerhaftes Einsetzen, das zu einer Fehlfunktion des Pumpsystems führen könnte, sicher verhindert wird.

Zur weiteren Sicherung der Schlauchkassette an der Rollenpumpe 1 kann schließlich der Verriegelungshebel 10 so weit gedreht werden, dass das Ende des Verriegelungshebels 10 über der Endfläche 27 der Rampe 26 an der Oberseite der Schlauchkassette 20 zu liegen kommt (in Fig. 4b gestrichelt angedeutet). In dieser Position rastet ein an der Rückseite des Verriegelungshebels 10 angeordnetes Druckstück auf der Endfläche 27, die als Rastfläche wirkt, ein. Da die Endfläche 27 bezüglich der Schlauchkassette 20 näherungsweise oberhalb der Druckmessmembran 34 angeordnet ist, beispielsweise zwischen einer Projektion der Druckmessmembran 34 und einer Projektion der Aussparung 35 auf die Oberseite der Schlauchkassette 20, wird die Schlauchkassette 20 im Bereich der Drucksensoren 6, 6' fest an die Verbindungsfläche 3 der Rollenpumpe 1 angedrückt, so dass die Druckmessmembran 34 mit einer vorbestimmten Kraft an den Drucksensor 6 angepresst wird. Hierdurch werden die Genauigkeit und die Reproduzierbarkeit der Druckmessung verbessert. Eine Verschiebung der Schlauchkassette 20 gegenüber der Verbindungsfläche 3 ist nach Verriegelung mit dem Verriegelungshebel 10 nicht mehr möglich.

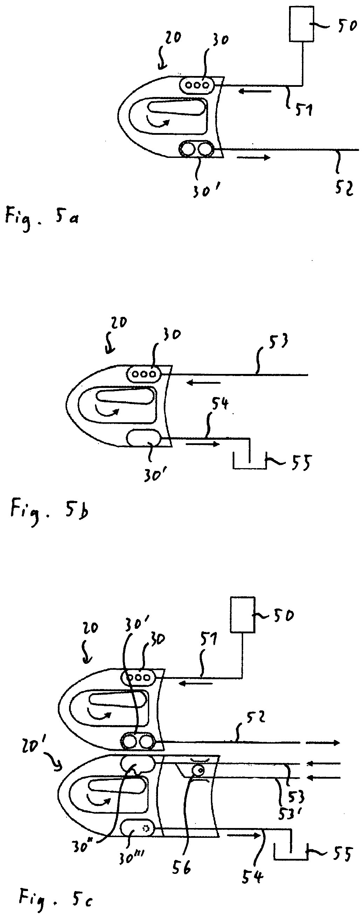

In den Figuren 5a bis 5c sind in schematischer Form beispielhaft mögliche Konfigurationen und Einsatzformen des erfindungsgemäßen Pumpsystems dargestellt. Die gekrümmten Pfeile stellen dabei die Drehrichtung des Pumpenkopfs und die geraden Pfeile die Fließrichtung der geförderten Flüssigkeit dar.

Gemäß Fig. 5a wird aus einem Versorgungsbehälter 50 eine Flüssigkeit durch die Schlauchkassette 20 zu einem Operationsgebiet gefördert, etwa um das Operationsgebiet selbst oder ein Sichtfenster einer Endoskopoptik zu spülen. Dabei dient der Zuleitungsschlauch als Versorgungsleitung 51, die mit dem Versorgungsbehälter 50 verbunden ist, und der Ableitungsschlauch als Spülleitung 52, die zum Operationsgebiet führt. Je nach Anwendungsfall kann es vorgesehen sein, dass der Druck am Ausgang der Schlauchkassette 20, d.h. am Zulauf zum Patienten, gemessen wird, um die Pumpfunktion entsprechend regeln zu können; hierfür weist das Verbindungselement 30' eine Druckmessmembran auf.

Wie in Fig. 5b gezeigt, kann die erfindungsgemäße Schlauchkassette auch zum Einsatz in einer Saugpumpe verwendet werden. Der Zuleitungsschlauch dient dabei als Saugleitung 53, die von einem Patienten zur Schlauchkassette 20 führt, und der Ableitungsschlauch als Abführungsleitung 54, durch die die abzuführende Flüssigkeit in einen Entsorgungsbehälter 55 befördert wird. Bei der Verwendung als Saugpumpe ist in der Regel keine Druckmessung erforderlich, so dass das Verbindungselement 30' ohne Druckmessmembran ausgebildet sein kann.

In Fig. 5c ist eine Kombination beider Anordnungen dargestellt, wobei eine Rollenpumpe zwei Pumpenköpfe aufweist, die jeweils mit einer Schlauchkassette 20, 20' verbindbar sind. Eine erste Schlauchkassette 20 ist als Spülpumpe in Betrieb, durch die Spülflüssigkeit von einem Versorgungsbehälter 50 zu einem Operationsgebiet gefördert wird. Die weitere Schlauchkassette 20' ist als Saugpumpe im Einsatz, wodurch abzusaugende Flüssigkeit aus einem Operationsgebiet in einen Sammelbehälter gefördert wird. Wie in Fig. 5c ferner gezeigt ist, können beispielsweise auch zwei Saugleitungen 53, 53' über einen Adapter 56 mit Klemmventilen abwechselnd an die Schlauchkassette 20' angeschlossen sei. Hierdurch kann etwa eine Absaugung sowohl durch eine Kanüle als auch von einem Shaver ermöglicht werden.

In Fig. 5c ist ferner eine mechanische Kodierung durch die Form des Verbindungselements 30" gezeigt, die bei einer entsprechenden Ausgestaltung der Verbindungsfläche 3 verhindert, dass die für eine Verwendung als Saugpumpe konfigurierte Schlauchkassette 20' als Spülpumpe eingesetzt wird. Hierdurch kann eine Fehlfunktion aufgrund einer Verwechslung einer Saug- und einer Spülkassette vermieden werden. Die Kodierung, aufgrund derer eine Steuerungseinrichtung der Rollenpumpe eine Verwendung der Schlauchkassette 20, 20' ermöglicht oder verhindert, kann auch mit Hilfe der oben beschriebenen Reflektionslichtschranken oder auch auf andere Weise, etwa durch drahtlose Datenübertragung (RFID), realisiert werden. Eine weitere Reflektionslichtschranke, die mit einem speziell ausgestalteten Verbindungselement 30'" zusammenwirkt, oder andere Identifikationsmittel können vorgesehen sein, um das Vorhandensein der Schlauchkassette 20' zu registrieren und einen Betrieb der Rollenpumpe bei Nichtvorhandensein der Schlauchkassette 20' zu verhindern. Hierdurch ist ein weiter erhöhtes Sicherheitsniveau erreichbar.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Rollenpumpe
- 2: Gehäusewand
- 3: Verbindungsfläche
- 4: Scheibe
- 5: Rolle
- 6, 6': Drucksensor
- 7, 7', 7": Reflektionslichtschranke
- 8: Haltesockel
- 9: Zunge
- 10: Verriegelungshebel
- 11: Rastkugel
- 12: Netzschalter
- 13: Bedienfeld
- 14: Anschluss
- 20, 20': Schlauchkassette
- 21: Erster Endbereich
- 22: Führungswand
- 23: Erhöhung
- 24: Zweiter Endbereich
- 25, 25': Ausbruch
- 26: Rampe
- 27: Endfläche
- 28: Öffnung
- 29: Pfeil
- 30, 30', 30", 30'": Verbindungselement
- 31, 31': Schlauchanschluss
- 32: Reflektionsfläche
- 33, 33': Ausnehmung
- 34: Druckmessmembran
- 35: Ausnehmung
- 36: Schlauchanschluss
- 37: Schräge
- 38, 38': Verbindungsrippe
- 39, 39': Verstärkungswulst
- 40: Zuleitungsschlauch
- 41: Schlauchsegment
- 42: Ableitungsschlauch
- 43: Rampe
- 44: Vertiefung
- 45: Feder
- 50: Versorgungsbehälter
- 51: Versorgungsleitung
- 52: Spülleitung
- 53, 53': Saugleitung
- 54: Abführungsleitung
- 55: Entsorgungsbehälter
- 56: Adapter

## Patentansprüche

1. Pumpsystem, insbesondere zur medizinischen Verwendung, umfassend eine Schlauchpumpe mit mindestens einem durch eine Gehäusewand (2) der Schlauchpumpe hindurch ragenden Pumpenkopf und mindestens eine mit der Schlauchpumpe durch Ansetzen an die Gehäusewand (2) koppelbare Schlauchkassette (20, 20') mit einem ersten Endbereich (21), in dem ein Schlauchsegment (41) zum Andrücken in einer Andruckrichtung an mindestens ein Pumpelement des Pumpenkopfs haltbar ist, und einem zweiten, vom ersten in der Andruckrichtung beabstandeten Endbereich (24), **dadurch gekennzeichnet, dass** die Schlauchpumpe ein feststehendes erstes Halteelement, mit dem die mindestens eine Schlauchkassette (20, 20') durch eine im Wesentlichen in Andruckrichtung verlaufende Bewegung verbindbar ist, zum formschlüssigen Halten des ersten Endbereichs (21) an der Gehäusewand (2) umfasst und ein feststehendes zweites Halteelement zum Halten des zweiten Endbereichs (24) der mindestens einen Schlauchkassette (20, 20') an der Gehäusewand (2) umfasst, wobei das zweite Halteelement und/oder die mindestens eine Schlauchkassette (20, 20') eine Schräge (37) zum Verschieben der Schlauchkassette (20, 20') in der Andruckrichtung beim Ansetzen an die Gehäusewand (2) aufweist.

2. Pumpsystem nach Anspruch 1, **dadurch gekennzeichnet, dass** die Schlauchkassette (20, 20') als Rahmen ausgebildet ist, der eine durchgehende Öffnung (28) umschließt, und dass das erste Halteelement als beim Ansetzen der Schlauchkassette (20, 20') an die Gehäusewand (2) die Öffnung (28) durchgreifende Haltezunge (9) ausgebildet ist, hinter die der erste Endbereich (21) der Schlauchkassette (20, 20') zum formschlüssigen Halten an der Gehäusewand (2) schiebbar ist.

3. Pumpsystem nach Anspruch 2, **dadurch gekennzeichnet, dass** die Haltezunge (9) von der Gehäusewand (2) weg weisend gekrümmt ausgebildet ist.

4. Pumpsystem nach Anspruch 2 oder 3, **dadurch gekennzeichnet, dass** das zweite Halteelement benachbart zum Pumpenkopf angeordnet ist und die Haltezunge (9) trägt.

5. Pumpsystem nach einem der Ansprüche 2 bis 4, **dadurch gekennzeichnet, dass** eine mit dem zweiten Halteelement zusammenwirkende Innenseite der Öffnung (28) der Schlauchkassette (20, 20') die Schräge (37) und das zweite Halteelement eine auf die Schräge (37) wirkende gefederte Raste aufweisen.

6. Pumpsystem nach Anspruch 5, **dadurch gekennzeichnet, dass** auf der Innenseite der Öffnung (28) der Schlauchkassette (20, 20') eine Rastvertiefung zum Eingreifen der gefederten Raste angeordnet ist.

7. Pumpsystem nach Anspruch 5 oder 6, **dadurch gekennzeichnet, dass** die Federkraft und der Federweg der gefederten Raste bemessen sind, um nach Ansetzen der Schlauchkassette (20, 20') an die Gehäusewand (2) eine Verschiebung der Schlauchkassette (20, 20') gegen die vom Schlauchsegment (41) ausgeübte elastische Rückstellkraft zu ermöglichen.

8. Pumpsystem nach einem der vorhergehenden Ansprüche, **dadurch gekennzeichnet, dass** die Schräge (37) als eine bogenförmig konvex gekrümmte Rampe ausgebildet ist.

9. Pumpsystem nach einem der vorhergehenden Ansprüche, **dadurch gekennzeichnet, dass** an der Gehäusewand (2) ein um eine näherungsweise senkrecht zur Gehäusewand (2) stehende Achse schwenkbarer Verriegelungshebel (10) angeordnet ist.

10. Pumpsystem nach Anspruch 9, **dadurch gekennzeichnet, dass** die Schlauchkassette (20, 20') auf ihrer Oberseite eine entlang einer Bahn eines Druckstücks des Verriegelungshebels (10) verlaufende ansteigende Rampe (26) aufweist.

11. Pumpsystem nach Anspruch 10, **dadurch gekennzeichnet, dass** die Schlauchkassette (20, 20') mindestens eine Druckmessmembran (34) zum Zusammenwirken mit einem Drucksensor (6, 6') der Schlauchpumpe aufweist und dass die Druckmessmembran (34), der Verriegelungshebel (10) und die Rampe (26) derart angeordnet sind, dass der Verriegelungshebel (10) in einem Endbereich der Rampe (26) die Schlauchkassette (20, 20') im Bereich der Druckmessmembran (34) an die Gehäusewand (2) andrückt.

12. Schlauchpumpe, insbesondere zur medizinischen Verwendung, mit mindestens einem durch eine Gehäusewand (2) der Schlauchpumpe hindurch ragenden Pumpenkopf, wobei mindestens eine Schlauchkassette (20, 20'), die einen ersten Endbereich (21), in dem ein Schlauchsegment (41) zum Andrücken in einer Andruckrichtung an mindestens ein Pumpelement des Pumpenkopfs haltbar ist, und einen zweiten, vom ersten in der Andruckrichtung beabstandeten Endbereich (24) aufweist, mit der Schlauchpumpe durch Ansetzen an die Gehäusewand (2) koppelbar ist, **dadurch gekennzeichnet, dass** die Schlauchpumpe ein feststehendes erstes Halteelement, mit dem die mindestens eine Schlauchkassette (20, 20') durch eine im Wesentlichen in Andruckrichtung verlaufende Bewegung verbindbar ist, zum formschlüssigen Halten des ersten Endbereichs (21) an der Gehäusewand (2) umfasst und ein feststehendes zweites Halteelement zum Halten des zweiten Endbereichs (24) der mindestens einen Schlauchkassette (20, 20') an der Gehäusewand (2) umfasst, wobei das zweite Halteelement eine Schräge (37) zum Verschieben der Schlauchkassette (20, 20') in der Andruckrichtung beim Ansetzen an die Gehäusewand (2) aufweist.

13. Schlauchkassette (20, 20'), insbesondere zur medizinischen Verwendung, die durch Ansetzen an eine Gehäusewand (2) einer Schlauchpumpe, die mindestens einen durch die Gehäusewand (2) hindurch ragenden Pumpenkopf aufweist, mit der Schlauchpumpe koppelbar ist, und die einen ersten Endbereich (21), in dem ein Schlauchsegment (41) zum Andrücken in einer Andruckrichtung an mindestens ein Pumpelement des Pumpenkopfs haltbar ist, und einen zweiten, vom ersten in der Andruckrichtung beabstandeten Endbereich (24) aufweist, **dadurch gekennzeichnet, dass** die Schlauchkassette (20, 20') eine Schräge (37) zum Verschieben der Schlauchkassette (20, 20') in der Andruckrichtung beim Ansetzen an die Gehäusewand (2) aufweist.

14. Verfahren zum Konfigurieren eines Pumpsystems, umfassend folgende Schritte:
- Bereitstellen einer Schlauchkassette (20, 20') mit einem in diese eingesetzten Schlauchsegment (41);
- Bereitstellen einer Schlauchpumpe, mit der die Schlauchkassette (20, 20') koppelbar ist, mit einem Gehäuse, das eine Gehäusewand (2) aufweist, durch die ein Pumpenkopf hinausragt;
- Ansetzen der Schlauchkassette (20, 20') an die Gehäusewand (2) derart, dass ein erster Endbereich (21) der Schlauchkassette (20, 20') in Berührung mit dem Gehäuse der Schlauchpumpe steht, während der zweite Endbereich (24) von diesem noch abgehoben ist;
- Einschieben des ersten Endbereichs (21) in einer Andruckrichtung in ein erstes Halteelement zur formschlüssigen Verbindung des ersten Endbereichs (21) mit der Schlauchpumpe bis zur Anlage des in die Schlauchkassette (20, 20') eingesetzten Schlauchsegments (41) an mindestens einem Pumpelement des Pumpenkopfs;
- Anschwenken der Schlauchkassette (20, 20') mit ihrem zweiten Endbereich (24) an die Gehäusewand (2) und Verschieben der Schlauchkassette (20, 20') in der Andruckrichtung zum Andrücken des Schlauchsegments (41) an das mindestens eine Pumpelement;
- Verriegeln der Schlauchkassette (20, 20') durch Drehen eines Verriegelungshebels (10), der auf die Vorderseite der Schlauchkassette (20, 20') drückt.

15. Verfahren nach Anspruch 14, wobei das Bereitstellen der Schlauchkassette (20, 20') die folgenden Schritte umfasst:
- Bereitstellen eines Schlauchsets mit einem Zuleitungsschlauch (40), einem Ableitungsschlauch (42), einem Schlauchsegment (41) und zwei Verbindungselementen (30, 30', 30", 30"'), wobei die Verbindungselemente (30, 30', 30", 30"') entsprechend der vorgesehenen Verwendung bei einem chirurgischen Eingriff ausgewählt sind;
- Verbinden des Zuleitungsschlauchs (40) und des Ableitungsschlauchs (42) über die Verbindungselemente (30, 30', 30", 30"') mit dem Schlauchsegment (41);
- Einsetzen des Schlauchsegments (41) und der Verbindungselemente (30, 30', 30", 30'") in die Schlauchkassette.

## Claims

1. Pump system, in particular for medical use, comprising a tubing pump, with at least one pump head protruding through a housing wall (2) of the tubing pump, and at least one tubing cassette (20, 20') which can be coupled to the tubing pump by being placed onto the housing wall (2) and which has a first end area (21), in which a tubing segment (41) can be held for pressing in a pressing direction onto at least one pump element of the pump head, and a second end area (24) spaced apart from the first end area in the direction of pressing, **characterized in that** the tubing pump comprises a stationary first holding element, to which the at least one tubing cassette (20, 20') can be connected by a movement extending substantially in the pressing direction, for form-fit retention of the first end area (21) on the housing wall (2), and a stationary second holding element for holding the second end area (24) of the at least one tubing cassette (20, 20') on the housing wall (2), wherein the second holding element and/or the at least one tubing cassette (20, 20') has a bevel (37) for sliding the tubing cassette (20, 20') in the pressing direction during placement onto the housing wall (2).

2. Pump system according to Claim 1, **characterized in that** the tubing cassette (20, 20') is designed as a frame which encloses a through-opening (28), and **in that** the first holding element is designed as a holding tongue (9) which engages through the opening (28) when the tubing cassette (20, 20') is placed onto the housing wall (2), behind which holding tongue (9) the first end area (21) of the tubing cassette (20, 20') can be pushed for the form-fit retention on the housing wall (2).

3. Pump system according to Claim 2, **characterized in that** the holding tongue (9) is designed curving away from the housing wall (2).

4. Pump system according to Claim 2 or 3, **characterized in that** the second holding element is arranged adjacent to the pump head and carries the holding tongue (9).

5. Pump system according to one of Claims 2 to 4, **characterized in that** an inner face of the opening (28) of the tubing cassette (20, 20') interacting with the second holding element has the bevel (37), and the second holding element has a sprung latch that acts on the bevel (37).

6. Pump system according to Claim 5, **characterized in that** a latch depression for the engagement of the sprung latch is arranged on the inner face of the opening (28) of the tubing cassette (20, 20').

7. Pump system according to Claim 5 or 6, **characterized in that** the spring force and the spring excursion of the sprung latch are such that, after placement of the tubing cassette (20, 20') onto the housing wall (2), they permit a movement of the tubing cassette (20, 20') counter to the elastic restoring force exerted by the tubing segment (41).

8. Pump system according to one of the preceding claims, **characterized in that** the bevel (37) is designed as a ramp that is convexly curved in an arc shape.

9. Pump system according to one of the preceding claims, **characterized in that** a locking lever (10) is arranged on the housing wall (2) and is pivotable about an axis lying approximately perpendicular to the housing wall (2).

10. Pump system according to Claim 9, **characterized in that** the tubing cassette (20, 20') has, on its upper face, an ascending ramp (26) extending along a path of a pressure piece of the locking lever (10).

11. Pump system according to Claim 10, **characterized in that** the tubing cassette (20, 20') has at least one pressure measurement membrane (34) for interaction with a pressure sensor (6, 6') of the tubing pump, and **in that** the pressure measurement membrane (34), the locking lever (10) and the ramp (26) are arranged in such a way that the locking lever (10) in an end area of the ramp (26) presses the tubing cassette (20, 20') onto the housing wall (2) in the area of the pressure measurement membrane (34).

12. Tubing pump, in particular for medical use, comprising at least one pump head protruding through a housing wall (2) of the tubing pump, wherein at least one tubing cassette (20, 20'), which has a first end area (21) in which a tubing segment (41) can be held for pressing in a pressing direction onto at least one pump element of the pump head, and a second end area (24), spaced apart from the first end area in the direction of pressing, can be coupled to the tubing pump by placement onto the housing wall (2), **characterized in that** the tubing pump comprises a stationary first holding element, to which the at least one tubing cassette (20, 20') can be connected by a movement extending substantially in the pressing direction, for the form-fit retention of the first end area (21) on the housing wall (2), and a stationary second holding element for holding the second end area (24) of the at least one tubing cassette (20, 20') on the housing wall (2), wherein the second holding element has a bevel (37) for sliding the tubing cassette (20, 20') in the pressing direction during placement onto the housing wall (2).

13. Tubing cassette (20, 20'), in particular for medical use, which can be placed onto a housing wall (2) of a tubing pump having at least one pump head protruding through the housing wall (2) and can thus be coupled to the tubing pump, and which has a first end area (21), in which a tubing segment (41) can be held for pressing in a pressing direction onto at least one pump element of the pump head, and a second end area (24) spaced apart from the first end area in the direction of pressing, **characterized in that** the tubing cassette (20, 20') has a bevel (37) for sliding the tubing cassette (20, 20') in the pressing direction during placement onto the housing wall (2).

14. Method for configuring a pump system, comprising the following steps:
- making available a tubing cassette (20, 20') with a tubing segment (41) inserted therein;
- making available a tubing pump to which the tubing cassette (20, 20') can be coupled, with a housing that has a housing wall (2) through which a pump head protrudes;
- placing the tubing cassette (20, 20') onto the housing wall (2) in such a way that a first end area (21) of the tubing cassette (20, 20') is in contact with the housing of the tubing pump, while the second end area (24) is still held away from the latter;
- pushing the first end area (21) in a pressing direction into a first holding element for form-fit connection of the first end area (21) to the tubing pump until the tubing segment (41) inserted into the tubing cassette (20, 20') bears on at least one pump element of the pump head;
- pivoting the tubing cassette (20, 20') with its second end area (24) onto the housing wall (2) and sliding the tubing cassette (20, 20') in the pressing direction in order to press the tubing segment (41) onto the at least one pump element;
- locking the tubing cassette (20, 20') by turning a locking lever (10), which presses onto the front face of the tubing cassette (20, 20').

15. Method according to Claim 14, wherein the making available of the tubing cassette (20, 20') comprises the following steps:
- making available a tubing set with a feed tubing (40), a withdrawal tubing (42), a tubing segment (41) and two connection elements (30, 30', 30'', 30'''), wherein the connection elements (30, 30', 30'', 30''') are chosen according to the intended use in a surgical intervention;
- connecting the feed tubing (40) and the withdrawal tubing (42) to the tubing segment (41) via the connection elements (30, 30', 30'', 30''');
- inserting the tubing segment (41) and the connection elements (30, 30', 30'', 30''') into the tubing cassette.

## Revendications

1. Système de pompe, en particulier pour usage médical, comprenant une pompe tubulaire avec au moins une tête de pompe saillant à travers une paroi de boîtier (2) de la pompe tubulaire, et comprenant au moins une cassette tubulaire (20, 20') pouvant être accouplée à la pompe tubulaire par application contre la paroi de boîtier (2), avec une première région d'extrémité (21) dans laquelle peut être retenu un segment tubulaire (41) destiné à presser dans une direction de pressage contre au moins un élément de pompe de la tête de pompe, et une deuxième région d'extrémité (24) espacée de la première dans la direction de pressage, **caractérisé en ce que** la pompe tubulaire comprend un premier élément de retenue fixe avec lequel l'au moins une cassette tubulaire (20, 20') peut être connectée par un déplacement s'étendant essentiellement dans la direction de pressage, afin de retenir par engagement par correspondance de formes la première région d'extrémité (21) contre la paroi de boîtier (2), et comprend un deuxième élément de retenue fixe pour retenir la deuxième région d'extrémité (24) de l'au moins une cassette tubulaire (20, 20') contre la paroi de boîtier (2), le deuxième élément de retenue et/ou l'au moins une cassette tubulaire (20, 20') présentant un biseau (37) pour déplacer la cassette tubulaire (20, 20') dans la direction de pressage lors de l'application contre la paroi de boîtier (2).

2. Système de pompe selon la revendication 1, **caractérisé en ce que** la cassette tubulaire (20, 20') est réalisée sous forme de cadre qui entoure une ouverture traversante (28), et **en ce que** le premier élément de retenue est réalisé sous forme de langue de retenue (9) s'engageant à travers l'ouverture (28) lors de l'application de la cassette tubulaire (20, 20') contre la paroi de boîtier (2), derrière laquelle langue de retenue la première région d'extrémité (21) de la cassette tubulaire (20, 20') peut être poussée de manière à être retenue par engagement par correspondance de formes contre la paroi de boîtier (2).

3. Système de pompe selon la revendication 2, **caractérisé en ce que** la langue de retenue (9) est réalisée sous forme courbe en s'écartant de la paroi de boîtier (2).

4. Système de pompe selon la revendication 2 ou 3, **caractérisé en ce que** le deuxième élément de retenue est adjacent à la tête de pompe et porte la langue de retenue (9).

5. Système de pompe selon l'une quelconque des revendications 2 à 4, **caractérisé en ce qu'**un côté intérieur de l'ouverture (28) de la cassette tubulaire (20, 20') coopérant avec le deuxième élément de retenue présente le biseau (37), et le deuxième élément de retenue présente un cliquet à ressort agissant sur le biseau (37).

6. Système de pompe selon la revendication 5, **caractérisé en ce qu'**un renfoncement d'encliquetage pour l'engagement du cliquet à ressort est disposé sur le côté intérieur de l'ouverture (28) de la cassette tubulaire (20, 20').

7. Système de pompe selon la revendication 5 ou 6, **caractérisé en ce que** la force de ressort et la course de ressort du cliquet à ressort sont dimensionnées de manière à permettre, après l'application de la cassette tubulaire (20, 20') contre la paroi de boîtier (2), un déplacement de la cassette tubulaire (20, 20') à l'encontre de la force de rappel élastique exercée par le segment tubulaire (41).

8. Système de pompe selon l'une quelconque des revendications précédentes, **caractérisé en ce que** le biseau (37) est réalisé sous forme de rampe de courbure convexe en forme d'arc.

9. Système de pompe selon l'une quelconque des revendications précédentes, **caractérisé en ce qu'**un levier de verrouillage (10) pouvant pivoter autour d'un axe approximativement perpendiculaire à la paroi de boîtier (2) est disposé au niveau de la paroi de boîtier (2).

10. Système de pompe selon la revendication 9, **caractérisé en ce que** la cassette tubulaire (20, 20') présente au niveau de son côté supérieur une rampe montante (26) s'étendant le long d'une trajectoire d'une pièce de pression du levier de verrouillage (10).

11. Système de pompe selon la revendication 10, **caractérisé en ce que** la cassette tubulaire (20, 20') présente au moins une membrane de mesure de pression (34) destinée à coopérer avec un capteur de pression (6, 6') de la pompe tubulaire et **en ce que** la membrane de mesure de pression (34), le levier de verrouillage (10) et la rampe (26) sont disposés de telle sorte que le levier de verrouillage (10), dans une région d'extrémité de la rampe (26), presse la cassette tubulaire (20, 20') dans la région de la membrane de mesure de pression (34) contre la paroi de boîtier (2).

12. Pompe tubulaire, en particulier pour usage médical, comprenant au moins une tête de pompe saillant à travers une paroi de boîtier (2) de la pompe tubulaire, au moins une cassette tubulaire (20, 20') qui présente une première région d'extrémité (21) dans laquelle un segment tubulaire (41) est retenu pour presser dans une direction de pressage contre au moins un élément de pompe de la tête de pompe, et une deuxième région d'extrémité (24) espacée de la première dans la direction de pressage, pouvant être accouplée à la pompe tubulaire par application contre la paroi de boîtier (2), **caractérisée en ce que** la pompe tubulaire comprend un premier élément de retenue fixe avec lequel l'au moins une cassette tubulaire (20, 20') peut être connectée par un déplacement s'étendant essentiellement dans la direction de pressage, afin de retenir par engagement par correspondance de formes la première région d'extrémité (21) contre la paroi de boîtier (2), et comprend un deuxième élément de retenue fixe pour retenir la deuxième région d'extrémité (24) de l'au moins une cassette tubulaire (20, 20') contre la paroi de boîtier (2), le deuxième élément de retenue présentant un biseau (37) pour déplacer la cassette tubulaire (20, 20') dans la direction de pressage lors de l'application contre la paroi de boîtier (2).

13. Cassette tubulaire (20, 20'), en particulier pour usage médical, qui peut être accouplée à la pompe tubulaire par application contre une paroi de boîtier (2) d'une pompe tubulaire qui présente au moins une tête de pompe saillant à travers la paroi de boîtier (2), et qui présente une première région d'extrémité (21) dans laquelle un segment tubulaire (41) peut être retenu pour presser dans une direction de pressage contre au moins un élément de pompe de la tête de pompe, et une deuxième région d'extrémité (24) espacée de la première dans la direction de pressage, **caractérisée en ce que** la cassette tubulaire (20, 20') présente un biseau (37) pour déplacer la cassette tubulaire (20, 20') dans la direction de pressage lors de l'application contre la paroi de boîtier (2).

14. Procédé de configuration d'un système de pompe, comprenant les étapes suivantes :
- fourniture d'une cassette tubulaire (20, 20') avec un segment tubulaire (41) inséré dans celle-ci ;
- fourniture d'une pompe tubulaire avec laquelle la cassette tubulaire (20, 20') peut être accouplée, avec un boîtier qui présente une paroi de boîtier (2) à travers laquelle sort une tête de pompe ;
- application de la cassette tubulaire (20, 20') contre la paroi de boîtier (2) de telle sorte qu'une première région d'extrémité (21) de la cassette tubulaire (20, 20') soit en contact avec le boîtier de la pompe tubulaire tandis que la deuxième région d'extrémité (24) est encore soulevée de celui-ci ;
- insertion de la première région d'extrémité (21) dans une direction de pressage dans un premier élément de retenue pour réaliser la connexion par engagement par correspondance de formes de la première région d'extrémité (21) à la pompe tubulaire jusqu'à ce que le segment tubulaire (41) inséré dans la cassette tubulaire (20, 20') vienne s'appliquer contre au moins un élément de pompe de la tête de pompe ;
- pivotement de la cassette tubulaire (20, 20') avec sa deuxième région d'extrémité (24) contre la paroi de boîtier (2) et déplacement de la cassette tubulaire (20, 20') dans la direction de pressage pour presser le segment tubulaire (41) contre l'au moins un élément de pompe ;
- verrouillage de la cassette tubulaire (20, 20') par rotation d'un levier de verrouillage (10) qui presse sur le côté avant de la cassette tubulaire (20, 20').

15. Procédé selon la revendication 14, dans lequel la fourniture de la cassette tubulaire (20, 20') comprend les étapes suivantes :
- fourniture d'un ensemble de tuyaux avec un tuyau d'alimentation (40), un tuyau de drainage (42), un segment tubulaire (41) et deux éléments de connexion (30, 30', 30'', 30'''), les éléments de connexion (30, 30', 30'', 30''') étant choisis en fonction de l'utilisation prévue dans le cas d'une intervention chirurgicale ;
- connexion du tuyau d'alimentation (40) et du tuyau de drainage (42) par le biais des éléments de connexion (30, 30', 30'', 30''') au segment tubulaire (41) ;
- insertion du segment tubulaire (41) et des éléments de connexion (30, 30', 30'', 30''') dans la cassette tubulaire.
